(19) Europäisches Patentamt / European Patent Office / Office européen des brevets

(11) **EP 2 044 096 B1**

(12) # FASCICULE DE BREVET EUROPEEN

(45) Date de publication et mention de la délivrance du brevet:
**16.06.2010 Bulletin 2010/24**

(21) Numéro de dépôt: **07803979.9**

(22) Date de dépôt: **05.07.2007**

(51) Int Cl.:
***C07H 15/08*** *(2006.01)* ***C11D 1/66*** *(2006.01)*
***C11D 1/72*** *(2006.01)* ***C11D 3/20*** *(2006.01)*
***C07C 43/13*** *(2006.01)* ***B01F 17/00*** *(2006.01)*
***A61K 8/39*** *(2006.01)*

(86) Numéro de dépôt international:
**PCT/FR2007/051600**

(87) Numéro de publication internationale:
**WO 2008/007003 (17.01.2008 Gazette 2008/03)**

(54) **NOUVEAUX DERIVES DE SUCRE, PROCEDE POUR LEUR PREPARATION ET LEUR UTILISATION COMME AGENTS TENSIOACTIFS**

NEUE ZUCKERDERIVATE, VERFAHREN FÜR IHRE ZUBEREITUNG UND IHRE VERWENDUNG ALS OBERFLÄCHENAKTIVE MITTEL

NEW SUGAR DERIVATIVES, METHOD FOR PREPARATION THEREOF, THEIR USE AS SURFACE-ACTIVE AGENTS

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **10.07.2006 FR 0652882**

(43) Date de publication de la demande:
**08.04.2009 Bulletin 2009/15**

(73) Titulaire: **SOCIETE D'EXPLOITATION DE PRODUITS POUR LES INDUSTRIES CHIMIQUES-SEPPIC**
**75321 Paris Cedex 07 (FR)**

(72) Inventeurs:
- **GUILBOT, Jérôme**
**F-81100 Castres (FR)**
- **ROLLAND, Hervé**
**F-81100 Castres (FR)**
- **MULLER, Daniel**
**F-94100 Saint-Maur (FR)**

(74) Mandataire: **Conan, Philippe Claude**
**L'Air Liquide SA,**
**75 Quai d'Orsay**
**75321 Paris Cedex 07 (FR)**

(56) Documents cités:
**DE-A1- 19 728 900** **FR-A1- 2 804 432**
**US-A- 5 849 679**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 044 096 B1

## Description

L'invention a pour objet de nouveaux agents tensioactifs issus de sucres.

Les agents de surface de la famille des alkyl polyglycosides et des alkényl polyglycosides sont largement utilisés aujourd'hui comme agents moussants, agents émulsionnants et/ou agents solubilisants, que ce soit dans des formulations cosmétiques ou pharmaceutiques ou dans des formulations à usage industriel.

Le brevet des Etats-Unis d'Amérique N° 5,849,679 divulgue des agents tensioactifs non ioniques comprenant un mélange d'isomères $\alpha$ et $\beta$ anomériques d'éthers d'un composé glycosidique avec un mono-éther de glycérol ou de polyglycérol et d'alcool en $C_1$-$C_{22}$ et plus particulièrement les composés suivants : le 1-O-n-décylglycéryl-D-glucopyranoside, le 1-O-n-hexylglycéryl-D-glucopyranoside, le 1-O-n-dodécyltriglycéryl-D-glucopyranoside, le 1-O-n-oléyltriglycéryl-D-glucopyranoside et le stéaryl polyglycéryl-D-glucopyranoside ;

La demande de brevet allemand publiée sous le numéro DE 197 28 900 divulgue le même type de tensioactifs non ioniques dérivés du glycérol que ceux divulgués dans le document précédent et plus particulièrement les composés suivants : le lauryl glycéryl glucoside et le (2-éthyl hexyl) glycéryl glucoside.

La demande de brevet français publiée sous le numéro 2 804 432 divulgue aussi des composés dérivés de polyglycéryl glucosides ainsi que leur utilisation comme agents tensioactifs.

Dans le cadre de leurs recherches sur la mise au point de nouveaux agents tensioactifs, les inventeurs ont développé de nouvelles structures glycosidiques qui ont des propriétés tensioactives.

C'est pourquoi selon un premier aspect, l'invention a pour objet une composition (C) comprenant, pour 100% de sa masse :

- de 0,5% à 30% d'un ou de plusieurs alcools de formule (IV) :

  R1-OH (IV)

  dans laquelle R1 représente un radical aliphatique saturé ou insaturé, linéaire ou ramifié, comportant de 8 à 36 atomes de carbone, éventuellement substitué avec un ou plusieurs groupes hydroxyle,
  et
- de 70% à 99,5% d'un ou de plusieurs composés de formule (I) choisis parmi :

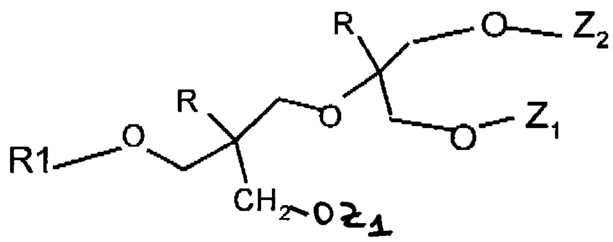

(Ib)

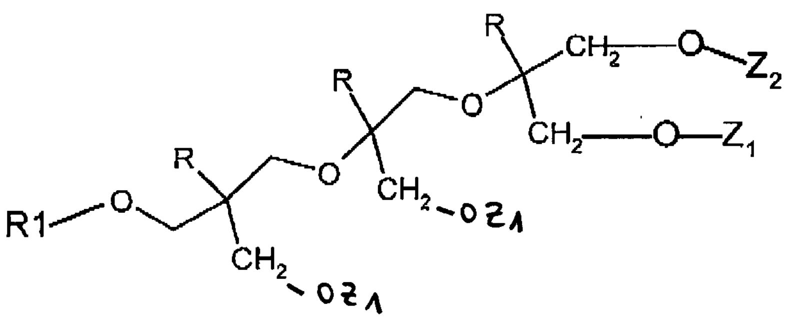

(Ic2)

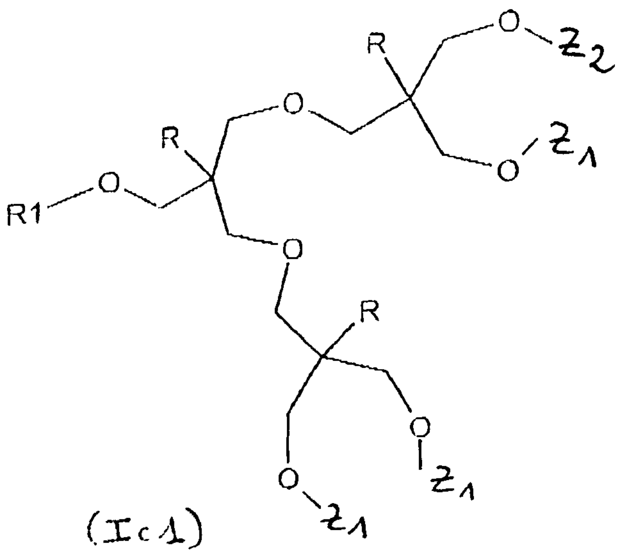

(Ic1)

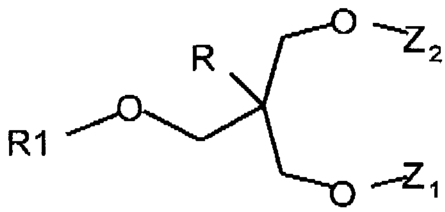

(Ia)

où R représente un radical alkyle linéaire ou ramifié comportant de un à huit atomes de carbone éventuellement substitué par un ou plusieurs radicaux hydroxy, R1 est tel que défini précédemment pour la formule (IV), au moins l'un des radicaux $Z_1$ ou $Z_2$ représente un radical :

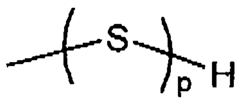

dans lequel -S- représente le reste divalent d'un sucre réducteur et p représente un nombre décimal supérieur ou égal à 1 et inférieur ou égal à 10 et l'autre des radicaux $Z_1$ ou $Z_2$ représente un atome d'hydrogène ou ledit radical :

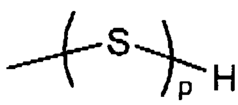

Par radical aliphatique saturé ou insaturé, linéaire ou ramifié comportant de 8 à 36 atomes de carbone, éventuellement substitué avec un ou plusieurs groupes hydroxyle, et/ou un ou plusieurs groupes oxo, on désigne pour R1, dans les formules (I) et (IV) telles que définies ci-dessus :

- les radicaux alkyle linéaires, par exemple les radicaux octyle, décyle, dodécyle, tétradécyle, hexadécyle, octadécyle, eicosyle ou docosyle ;
- les radicaux issus des isoalcanols de formule (A) :

$$(CH_3)(CH_3)CH\text{-}(CH_2)_m\text{-}CH_2\text{-}OH \quad (A)$$

dans laquelle m représente un nombre entier compris entre 4 et 18, par exemple les radicaux isooctyle, isodécyle, isododécyle, isotétradécyle, isohexadécyle ou isooctadécyle ;

- le radical 2-éthyl hexyle ou les radicaux alkyles ramifiés issus des alcools de Guerbet de formule (B) :

$$CH(C_sH_{2s+1})(C_tH_{2t+1})\text{-}CH_2\text{-}OH \quad (B)$$

dans laquelle t est un nombre entier compris entre 4 et 18, s est un nombre entier compris entre 2 et 16 et la somme s + t est supérieure ou égale à 8, par exemple les radicaux 2-éthyl décyle, 2-butyl octyle, 2-éthyl dodécyle, 2-butyl décyle, 2-hexyl octyle, 2-éthyl tétradécyle, 2-butyl décyle, 2-hexyl octyle, 2-éthyl hexadécyle, 2-butyl tétradécyle, 2-hexyl dodécyle, 2-octyl décyle, 2-hexyl décyle, 2-octyl dodécyle, 2-décyl tétradécyle, 2-dodécyl hexadécyle, 2-tétradécyl octadécyle, 2-tétradécyl eicosyle, 2-hexadécyl octadécyle ou 2-hexadécyl eicosyle ; ou les radicaux issus des homologues d'alcools de Guerbet, par exemple le radical 2-propyl heptyle.

- les radicaux linéaires insaturés tels que les radicaux undécènyle, docécènyle, tétradécènyle, hexadécènyle, octadécènyle, octadécadiènyle, octadécatriényle, octadécatétraènyle, eicosènyle, eicosadiènyle ou docosènyle, par exemple les radicaux insaturés 10-undécènyle, 4-dodécènyle, 5-dodécènyle, oléyle, isooléyle, linoléyle, linolényle ou 10, 13-eicosadiènyle ;
- les radicaux aliphatiques saturés ou insaturés, linéaires ou ramifiés, comportant de 8 à 36 atomes de carbone substitués par 1 ou deux groupes hydroxy, tels que les radicaux hydroxy hexadécyle, hydroxy octadécyle, dihydroxy docosyle ou dihydroxy-octadécyle, par exemple, les radicaux 3-hydroxy hexadécyle, l'acide 4-hydroxy hexadécyle, l'acide 11-hydroxy hexadécyle, l'acide 16-hydroxy hexadécyle, 12-hydroxy stéaryle ou 8,9-dihydroxy stéaryle.

Par radical allyle linéaire ou ramifié comportant de un à huit atomes de carbone éventuellement substitués par un ou plusieurs radicaux hydroxy ou un ou plusieurs, on désigne notamment pour R dans la formule (1) telle que définie ci-dessus, les radicaux méthyle, éthyle, propyle, isopropyle, butyle, isobutyle, pentyle, isopentyle, hexyle, isohexyle, heptyle isoheptyle, octyle, isooctyle, hydroxyméthyle ou 2-hydroxyéthyle.

Par reste d'un sucre réducteur, on désigne principalement pour -S- dans la formule (I) telle que définie ci-dessus, les restes du glucose, du saccharose, du fructose, de l'idose, du galactose, du maltose, du maltotriose, du lactose, du cellobiose, du mannose, du xylose, de l'arabinose, du ribose, du dextrane ou du tallose.

Dans la formule (I) telle que définie ci-dessus, p est un nombre décimal, qui représente le degré moyen de polymérisation du reste S.

Lorsque p est un nombre entier, $(S)_p$ est le reste polymérique de rang p du reste S.

Lorsque p est un nombre décimal, la formule (I) représente un mélange de composés $a_1$ [(Q)(S)] + $a_2$ [(Q)$(S)_2$]$^+$ $a_3$ [(Q)$(S)_3$] +...+ $a_q$ [(Q)$(S)_q$] dans lesquels Q représente le groupe lié au(x) reste(s) de sucre S de la formule (I) avec q représentant un nombre entier compris entre 1 et 10 et dans les proportions molaires $a_1$, $a_2$, $a_3$,... $a_q$ telles que :

$$\sum_{q=1}^{q=1} a_p = 1 \; ; \; a_1 > 0$$

Selon un premier aspect particulier de la présente invention, dans la formule (IV), R1 représente un radical choisi parmi les radicaux octyle, décyle, dodécyle, tétradécyle, hexadécyle, octadécyle, eicosyle, 2-éthyl hexyle, 2-butyl octyle, 2-butyl décyle, 2-hexyl octyle, 2-hexyl décyle, 2-hexyl dodécyle, 2-octyl décyle, 2-octyl dodécyle, 2-décyl tétradécyle, isododécyle, isotétradécyle, isohexadécyle, isooctadécyle, 10-undécènyle, oléyle, isooléyle, linoléyle, linolényle ou 10,13-eicosadiènyle, 12-hydroxy stéaryle ou le 2-propyl heptyle.

Selon un deuxième aspect particulier de la présente invention, dans la formule (I), -S- représente un reste divalent du glucose ou du xylose.

Selon un troisième aspect particulier de la présente invention, dans la formule (I), p est compris entre 1,005 et 5 et plus particulièrement entre 1,05 et 2.

Selon un quatrième aspect particulier de la présente invention, dans la formule (I), R est choisi parmi les radicaux méthyle, éthyle, propyle, butyle, pentyle, hexyle, heptyle, octyle et hydroxyméthyle.

L'invention a aussi pour objet, un procédé de préparation d'une composition (C) telle que définie précédemment, comprenant les étapes successives suivantes :

Une étape a) de réaction d'un alcool ou d'un mélange d'alcools de formule (IV)

R1-OH (IV)

dans laquelle R1 représente un radical aliphatique saturé ou insaturé, linéaire ou ramifié, comportant de 8 à 36 atomes de carbone, éventuellement substitué avec un ou plusieurs groupes hydroxyle ou d'un mélange d'alcools de formule (IV), avec un oxétane- 3, 3 - disubstitué de formule (V) :

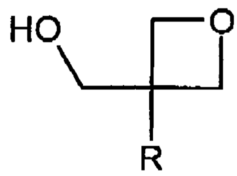

(V)

dans laquelle R représente un radical alkyle linéaire ou ramifié comportant de un à huit atomes de carbone éventuellement substitué par un ou plusieurs radicaux hydroxy, conduisant à un mélange (M) comprenant l'alcool de formule (IV) n'ayant pas réagi et un ou plusieurs composés choisis parmi les composés de formules (IIa), (IIb), ($IIc_1$) ou ($IIc_2$) :

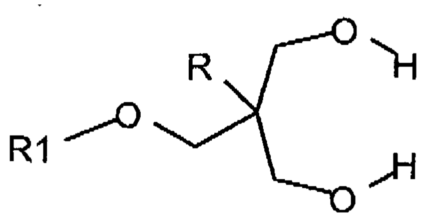

(IIa)

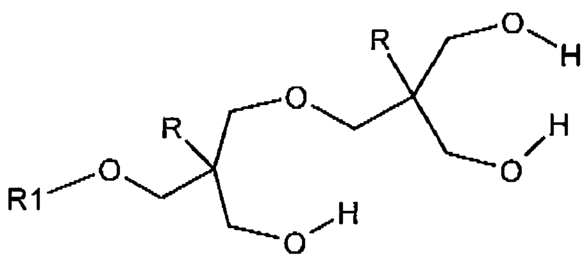

(IIb)

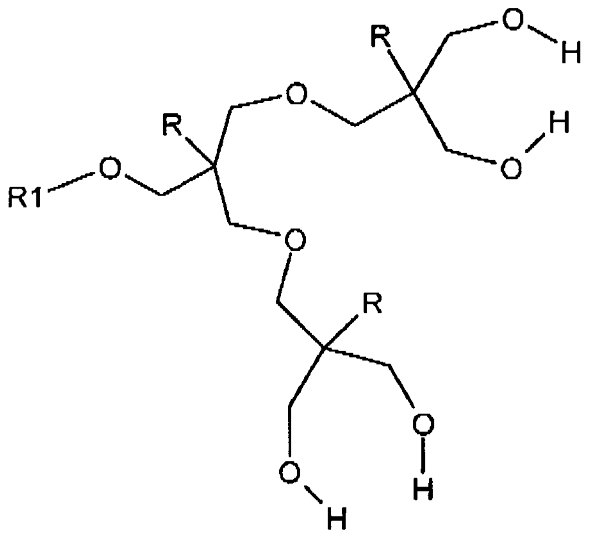

(IIc1)

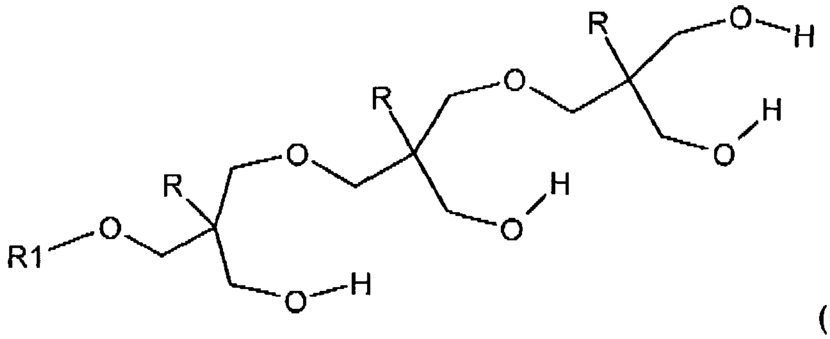

(IIc2)

dans lesquelles R et R1 sont tels que définis précédemment;

Une étape b) de réaction dudit mélange (M) avec un sucre réducteur de formule (III) :

S-H (III)

pour obtenir ladite composition tensioactive (C).

Dans le procédé tel que défini ci-dessus, l'étape (a) est généralement effectuée en présence d'un acide de Lewis comme catalyseur ; on peut citer par exemple, le trifluorure de bore stabilisé dans l'éther diéthylique ($BF_3.Et_2O$), le tétrachlorure d'étain ($SnCl_4$), le tétrachlorure de titane ($SnTi_4$) ou le trichlorure d'aluminium ($AlCl_3$)

Certains composés de formule (V) sont des composés connus, Le 3-éthyl 3-(hydroxyméthyl) oxétane de formule (V) (ou TMP oxétane) est un produit commercial identifié sous les numéros RN 3047-32-3 et EINECS 221-54-0.

Dans le procédé tel que défini ci-dessus, l'étape (b) est généralement effectuée en présence d'un acide fort comme catalyseur, par exemple l'acide sulfurique, l'acide chlorhydrique, l'acide phosphorique, acide méthanesulfonique, l'acide p-toluènesulfonique, l'acide trifluoro-méthanesulfonique ou l'acide hypophosphoreux.

La composition (C) objet de la présente invention, lorsqu'elle est obtenue par le procédé tel que défini précédemment, peut comprendre en outre jusqu'à 5% de sa masse de produits résiduels principalement le sucre réducteur de formule (III), le dérivé de l'oxétane de formule (V) et/ou des composés de formule (VI) :

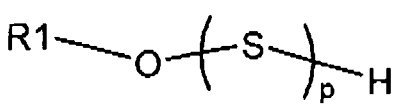

(VI)

dans laquelle R1, S et p sont tels que définis précédemment, et d'eau.

Selon un aspect particulier du procédé tel que défini ci-dessus, il comprend en outre une étape (c) de déshydratation d'un composé de formule (VII) :

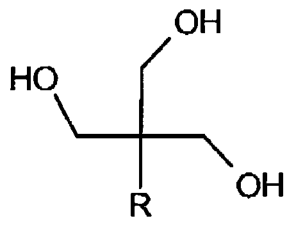

(VII)

dans laquelle R est tel que défini précédemment, pour obtenir le composé de formule (V).

L'étape (c) telle que définie ci-dessus, se compose généralement d'une étape initiale de réaction du composé de formule (VII) avec un carbonate de dialkyle, par exemple le carbonate de diméthyle, le carbonate de diéthyle ou le carbonate du dibutyle, pour obtenir un carbonate cyclique du dérivé de formule (VII), suivie d'une décarboxylation dudit carbonate cyclique pour obtenir l'oxétane disubstitué de formule (V).

Comme exemples de composés de formule (VII) connus, il y a le triméthylolpropane (TMP) (R représentant un radical éthyle) ou le pentaérythritol (R représentant un radical hydroxyméthyle).

L'invention a aussi pour objet l'utilisation d'une composition (C) telle que définie précédemment, comme agent tensioactif et plus particulièrement, comme agent moussant, agent émulsionnant, agent mouillant, agent dispersant ou agent détergent.

L'invention a enfin pour objet une composition cosmétique ou pharmaceutique caractérisée en ce qu'elle comprend comme agent tensioactif, une composition (C), telle que définie précédemment.

La partie expérimentale suivante illustre l'invention sans toutefois la limiter.

**Exemple 1 Préparation d'une composition C1 pour laquelle dans les formules (I) et (IV), R1 représente le radical octadécyle et dans la formule (I) R représente le radical éthyle et S représente le reste du glucose.**

Etape a) : Ouverture du TMP oxétane par l'octadécanol :

Le TMP oxétane est le composé de formule (V) tel que définie précédemment, dans laquelle R représente le radical éthyle. Cette première étape est réalisée à 120°C en présence d'une quantité catalytique d'acide de Lewis ($BF_3$-$Et_2O$). Deux stoechiométries différentes en TMP oxétane ont été étudiées (essais 1 et 2), la première impliquant une quantité équimolaire par rapport à l'octadécanol et la seconde impliquant un excès de 3 équivalents molaires toujours par rapport à l'octadécanol. Les conditions opératoires sont les suivantes : Fonte de l'octadécanol à ~120°C sous atmosphère d'azote, ajout de 0,5% de $BF_3.Et_2O$ (par rapport à la masse totale des réactifs) sous agitation mécanique, puis ajout progressif (en environ 180 minutes) du TMP oxétane, maintien à 120°C pendant 90 à 180 minutes, puis refroidissement. Les caractéristiques des mélanges (M1) et (M2) obtenus, sont rassemblées dans le tableau 1a ci-dessous ($T_A$ : Température ambiante ; IA : Indice d'acide ; IOH : Indice d'hydroxyle).

**Tableau 1a**

| | Essai 1 (M1) | Essai 2 (M2) |
|---|---|---|
| Stoechiométrie TMP oxétane / C-18 OH | 1 / 1 | 3 / 1 |
| Aspect visuel à TA | Solide blanc | Solide blanc |
| pH à1 % dans eau - isopropanol (50/50) | 5,0 | 4,9 |
| Eau (% massique) - Norme NFT 73201 | 0,01 | 0,03 |
| Point de fusion (°C) - Banc Kofler | < 44 | < 44 |
| IA (mg KOH/g) - Norme NFT 60-204 | 2,2 | 1,4 |
| IOH (mg KOH/g) - Norme USP (XXI NF XVI January 1-1985) | 285,6 | 351,3 |

(suite)

| | Essai 1 (M1) | Essai 2 (M2) |
|---|---|---|
| Octadécanol - (% massique) | 32,0 | 12,3 |
| TMP oxétane - (% massique) | 0,4 | 3,4 |
| Monoadduit (composé de formule (IIa) avec R1 : octadécyle) (% massique) | 27,8 | 18,2 |
| Diadduit (composé de formule (IIb) avec R1 = octadécyle, R : éthyle) - (% massique) | 15,9 | 15,5 |
| Triadduit (composé de formule (IIc) avec R1 = : octadécyle, R : éthyle)- (% massique) | 9,7 | 14,7 |

D'une manière générale, les résultats mettent en évidence une bonne réactivité entre l'octadécanol et le TMP oxétane. Théoriquement, l'indice d'hydroxyle du milieu réactionnel ne doit pas varier lors de la réaction et les valeurs finales mesurées sont cohérentes avec celles calculées (Mélange M1 : 285,6 mg KOH/g pour une valeur théorique de 289,2 mg KOH/g ; Mélange M2 : 351,3 mg KOH/g pour une valeur théorique de 361,8 mg KOH/g). Les dosages quantitatifs d'octadécanol résiduel montrent respectivement que, dans les essais 1 et 2, respectivement environ 54% et 72% de l'octadécanol de départ a été consommé lors de la réaction. Les dosages quantitatifs du TMP oxétane résiduel montrent respectivement que, dans les essais 1 et 2, plus de 98 % et 94 % de TMP oxétane de départ a réagi. Finalement, la distribution des différents adduits, établie par chromatographie en phase gazeuse, après une normalisation à 100%, montre que l'introduction d'un excès de TMP oxétane entraîne une consommation plus importante en octadécanol et favorise le phénomène de polymérisation des motifs TMP (n plus élevé).

Etape b): Acétalisation des adduits TMP oxétane 1 octadécanol avec le glucose:

Le protocole retenu est le suivant : Fonte des mélanges M1 et M2 à environ 100°C à pression atmosphérique et sous atmosphère d'azote, puis ajout, sous agitation mécanique, d'un équivalent molaire de glucose anhydre (la quantité est calculée à partir du nombre de mole d'octadécanol introduit à la première étape), ajout de 1% d'acide sulfurique à 98% (% massiques exprimés par rapport à la masse de glucose introduite), mise sous vide progressif [environ 30 $10^2$ Pa (30 mbars)] et acétalisation avec le glucose pendant 6 heures à environ 100°C ; refroidissement jusqu'à 80°C et neutralisation par ajout d'une solution de lessive de soude à 30% pour atteindre un pH proche de 7 ; filtration à chaud sur une plaque filtrante de porosité de 4 micromètres et conditionnement. Les caractéristiques analytiques des deux compositions (C1) et (C2) obtenues sont rassemblées dans le tableau 1b ci-dessous.

**Tableau 1b**

| Analyses | Composition C1 | Composition C2 |
|---|---|---|
| Produit de départ | Mélange M1 | Mélange M2 |
| Aspect visuel à $T_A$ | Solide blanc | Solide beige |
| pH à 1 % dans eau - éthanol (50/50) | 7,8 | 7,6 |
| Eau (%) - NFT 73201 | 0,41 | 0,42 |
| Couleur sur produit fondu - NFT 20-030 (vcs) | 2,0 | Non mesurable |
| Point de fusion (°C) Banc Kofler | ~ 50°C | 55 - 65°C |
| IA (mg KOH/g) - NFT 60-204 | 2,0 | 1,0 |
| IOH (mg KOH/g) - USP XXI NF XVI January 1-1985 | 393,9 | 412,8 |
| Octadécanol (% massique) | 24,2 % | 9,0 % |
| TMP oxétane (% massique) | < 0,05 % | < 0,05 |
| Glucose (% massique) | 0,2 % | < 0,1 |
| Composés de formule (VI) R1 : octadécyle ; S : glucose (% massique) | 3,0 % | 1,0 % |
| Composés de formule (Ia) R1 octadécyle ; S : glucose ; R : éthyle (% massique) | 3,6 % | 1,6 % |

(suite)

| Analyses | Composition C1 | Composition C2 |
|---|---|---|
| Composés de formule (Ib ou Ic) R1 : octadécyle ; S : glucose ; R : éthyle (% massique) | > 65% | > 85% |

L'augmentation significative de l'indice d'hydroxyle au cours des réactions indique une réactivité satisfaisante entre le glucose et les adduits « octadécanol + TMP ».

**Exemple 2 Préparation de deux compositions C3 et C4 pour lesquelles dans les formules (I) et (IV), R1 représente le radical octadécyle et hexadécyle dans la formule (I) R représente le radical éthyle et S représente le reste du glucose**.

On met en oeuvre les deux étapes développées à l'exemple précédent en utilisant à la place de l'octadécanol, de l'alcool cétéarylique (mélange à 50/50 molaire d'hexadécanol et d'octadécanol) sur deux essais dans des rapports stoechiométriques glucose / alcool de formule (IV) TMP oxétane. Les caractéristiques analytiques des deux compositions (C3) et (C4) obtenues sont rassemblées dans le tableau 2 ci-dessous.

**Tableau 2**

| Analyses | Composition C3 | Composition C4 |
|---|---|---|
| Produit de départ<br>Stoechiométrie molaire alcool de formule (IV) / TMP oxétane (formule (V)) | Mélange M3<br>1 / 2,5 | Mélange M3<br>1 / 2,5 |
| Stoechiométrie molaire glucose / alcool de formule (IV) | 0,5 / 1 | 1 / 1 |
| Aspect visuel à $T_A$ | Solide beige | Solide jaune |
| pH à 1 % dans eau - isopropanol | 6,6 | 6,0 |
| Eau (%)-NFT 73201 | 0,17 | 0,27 |
| Couleur sur produit fondu - NFT 20-030 (VCS) | 2,0 | 2 |
| Point de fusion (°C) Banc Kofler | < 44°C | < 44°C |
| IA (mg KOH/g) - NFT 60-204 | 1,3 | 1,3 |
| IOH (mg KOH/g) - USP XXI NF XVI January 1-1985 | 395,1 | 427,5 |
| Alcool cétéarylique (C16 / C18) (% massique) | 10, 6% | 9,8% |
| TMP oxétane (% massique) | < 0,01% | < 0,01% |
| Glucose (% massique) | < 0,1% | 0,4% |
| Composés de formule (VI) R1 : cétéaryle ; S : glucose (% massique) | 0,8% | 1.0% |
| Composés de formule (I) R1 : cétéaryle ; S : glucose ; R : éthyle, (% massique) | > 85% | > 85% |
| | | |

**Exemple 3 - Evaluation des propriétés émulsionnantes :**

3.1- Cas des émulsions Eau dans Huile:

Trois types d'huile ont été utilisés : une huile paraffinique (MARCOL™ 52), une huile polaire (Triglycéride 5545 en C-8/10) et une huile aromatique (SOLVESSO™ 100). Les deux formulations retenues sont :

- 20% d'huile, 2% de composition tensioactive et 78% d'eau de ville + 1% $MgSO_4$,
- 50% d'huile, 5% de composition tensioactive et 45% d'eau de ville + 1% de $MgSO_4$.

Le protocole de mis en en émulsion comprend les étapes suivantes :

1 - Mélange de l'huile et de la composition tensioactive, avec homogénéisation à la spatule, dans un bêcher de 400 ml forme haute, placé dans un bain-marie à 80°C ;
2 -Ajout de la phase aqueuse (eau de ville + 1% de $MgSO_4$ sec (2% de $MgSO_4$ heptahydrate)) à 80°C ;
3 - Cisaillement du mélange au Silverson™ (8000 tr/min pendant 4 min ; rotor/stator le plus bas possible dans le bécher et faire tourner doucement le bécher) et,
4 - Refroidissement jusqu'à température ambiante avec légère agitation pour obtenir 200 g d'émulsion.

Les résultats de stabilité à température ambiante ($T_A$) et à 40°C sont rassemblés dans le tableau 3 ci-dessous. Les cas où les mélanges ne restent pas totalement émulsionnés au bout de 24 heures à température ambiante n'ont pas été retenus.

**Tableau 3.**

| **Composition** | Huile | formulation : Composition Huile / (Eau+$MgSO_4$) | Observations | | |
|---|---|---|---|---|---|
| | | | t=1 jour | t = 13 jours (mélange M1 et composition C2)<br>t = 42 jours (composition C1 et mélange M2) | |
| | | | Température Ambiante | Température Ambiante | 40°C |
| **Mélange M1** | Triglycéride 5545 | 5/50/45 | Em.:100% | Em.: 93% | Em.: 71% |
| **Mélange M1** | MARCOL™ 52 | 5/50/45 | Em.: 100% | Em.: 80% | Em.: 51% |
| **Mélange M2** | MARCOL™ 52 | 5/50/45 | Em.: 100% | Em.: 90% | Em.:76% |
| **Composition C1** | Triglycéride 5545 | 2/20/78 | Em:100% | Em.:100% | Em.:47% |
| **Composition C1** | Triglycéride 5545 | 5/50/45 | Em.:100% | Em.:100% | Em.: 90% |
| **Composition C2** | SOLVESSO™ 100 | 5/50/45 | Em.:100% | Em.:38% | 3 phases |
| Em. : correspond au volume de phases émulsionné par rapport à la masse initiale préparée ; en fait Em. 93% signifie qu'un volume représentant 7 % du volume initial d'émulsion a déphasé. | | | | | |

Les stabilités observées montrent un pouvoir émulsionnant des compositions selon l'invention à température ambiante et à J1 pour les phases grasses étudiées. C1 possède un bon pouvoir émulsionnant pour préparer des émulsions eau - huile avec le triglycéride 5545.

<u>3.2- Cas des émulsions Huile dans Eau:</u>

Dans le cas des émulsions huile dans Eau, deux types d'huile ont été utilisés : une huile paraffinique (MARCOL™ 52) et une huile polaire (Triglycéride 5545 en C8/C10). Les formulations retenues sont identiques à celles précédemment décrites en utilisant de l'eau de ville exempte de $MgSO_4$.
Le protocole de mise en émulsion comprend les étapes suivantes :

1 - Mélange de l'eau de ville et de la composition tensioactive à 80°C,
2 - Ajout de l'huile et agitation à la spatule,
3 - Cisaillement du mélange au Silverson (8000 tr/min pendant 4 min ; rotor/stator le plus bas possible dans le bécher et faire tourner doucement le bécher) et,
4 - Refroidissement jusqu'à température ambiante avec légère agitation pour obtenir 200 g d'émulsion,

Les résultats de stabilité à température ambiante et à 40°C sont rassemblés dans le tableau 4 ci-dessous. Les cas où les mélanges ne restent pas totalement émulsionnés au bout de 24 heures à température ambiante n'ont pas été retenus.

Tableau 4

| **Composition** | Huile | Formulation : Composition / Huile / Eau | Observations | | |
|---|---|---|---|---|---|
| | | | t = 1 jour | t = 13 jours (composition C2)<br>t = 42 jours (composition C1 et MONTANOV™ 68) | |
| | | | **$T_A$** | **$T_A$** | **40°C** |
| **MONTANOV™ 68** | Triglycéride 5545 | 2/20/78 | Em.:100% | Em.:100% | Em.:100% |
| **MONTANOV™ 68** | Triglycéride 5545 | 5/50/45 | Em.:100% | Em.:100% | Em.:100% |
| **MONTANOV™ 68** | Triglycéride 5545 | 0,8 / 21,2 / 78 | Em.:66% | Déphasage | Déphasage |
| **MONTANOV™ 68** | Triglycéride 5545 | 0,3 / 21,7 78 | / Em.: 67% | Déphasage | Déphasage |
| **Composition C1** | Triglycéride 5545 | 2/20/78 | Em.:100% | Em.:100% | Em.:100% |
| **Composition C1** | Triglycéride 5545 | 5/50/45 | Em.: 100% | Em.: 100% | Em.: 100% |
| **Composition C1** | MARCOL™ 52 | 2/20/78 | Em.: 100% | Em.: 100% | Em.: 87% |
| **Composition C2** | Triglycéride 5545 | 2/20/78 | Em.:100% | Em.: 100% | Em.:100% |
| **Composition C2** | Triglycéride 5545 | 5/50/45 | Em.: 100% | Em.:100% | Em.:100% |
| **Composition C2** | MARCOL™ 52 | 2/20/78 | Em.: 100% | Em.:100% | Em.:88% |
| **Composition C2** | MARCOL™ 52 | 5/50/45 | Em.:100% | Em.:79% | 3 phases |

Globalement, les glucosides d'adduit « octadécanol + TMP » présentent des pouvoirs émulsionnants huile dans eau H/E remarquables en comparaison avec le Montanov™ 68 (mélange de composés de formule (IV) et (VI) avec R1 en C16-C18).

**Revendications**

**1.** Composition (C) comprenant pour 100% de sa masse :

- de 0,5% à 30% d'un ou de plusieurs alcools de formule (IV) :

R1-OH (IV)

dans laquelle R1 représente un radical aliphatique saturé ou insaturé, linéaire ou ramifié, comportant de 8 à 36 atomes de carbone, éventuellement substitué avec un ou plusieurs groupes hydroxyle.
et
- de 70% à 99,5% d'un ou de plusieurs composés de formule (I) choisis parmi :

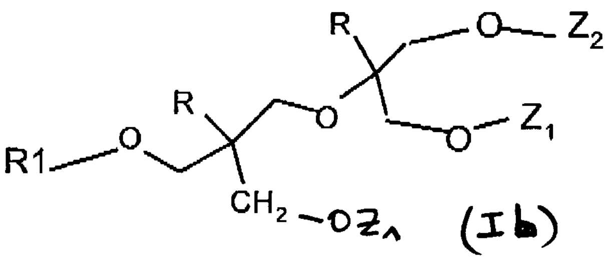

(Ib)

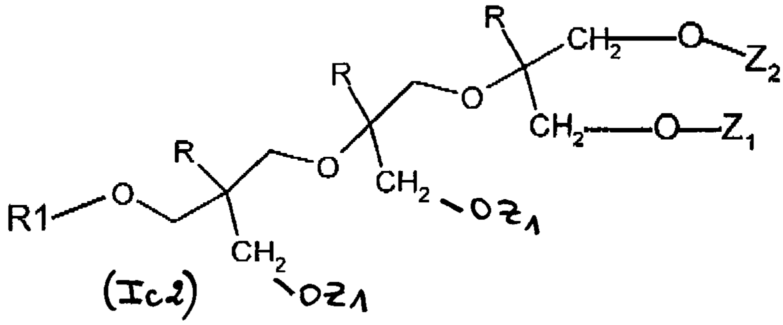

(Ic2)

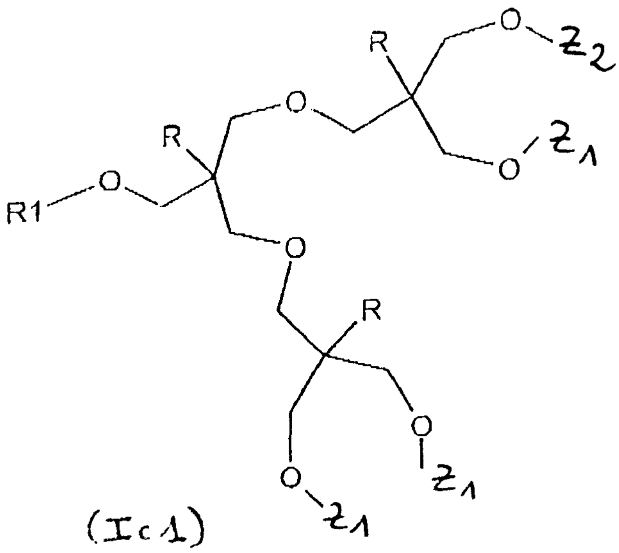

(Ic1)

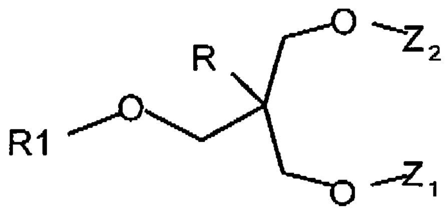

(Ia)

où R représente un radical alkyle linéaire ou ramifié comportant de un à huit atomes de carbone éventuellement substitué par un ou plusieurs radicaux hydroxy, R1 est tel que défini précédemment pour la formule (IV), au moins l'un des radicaux $Z_1$ ou $Z_2$ représente un radical :

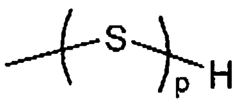

dans lequel -S- représente le reste divalent d'un sucre réducteur et p représente un nombre décimal supérieur ou égal à 1 et inférieur ou égal à 10 et l'autre des radicaux $Z_1$ ou $Z_2$ représente un atome d'hydrogène ou ledit

radical :

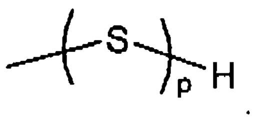

2. Composition (C) telle que définie à la revendication 1, pour laquelle R1 représente un radical choisi parmi les radicaux otyle, décyle, dodécyle, tétradécyle, hexadécyle, octadécyle, eicosyle, 2-éthyl hexyle, 2-butyl octyle, 2-butyl décyle, 2-hexyl octyle, 2-hexyl décyle, 2-hexyl dodécyle, 2-octyl décyle, 2-octyl dodécyle, 2-décyl tétradécyle, isododécyle, isotétradécyle, isohexadécyle, isooctadécyle, 10-undécènyle, oléyle, isooléyle, linoléyle, linolényle ou 10,13-eicosadiènyle, 12-hydroxy stéaryle et 2-propyl heptyle.

3. Composition (C) telle que définie à l'une des revendications 1 ou 2, où -S- représente un reste divalent du glucose.

4. Composition (C) telle que définie à l'une des revendications 1 ou 2, pour laquelle dans la formule (I), S représente un reste divalent du xylose.

5. Composition (C) telle que définie à l'une des revendications 1 à 4, pour laquelle, p est compris entre 1,005 et 5 et plus particulièrement entre 1,05 et 2.

6. Composition (C) telle que définie à l'une des revendications 1 à 4, pour laquelle, R est choisi parmi les radicaux méthyle, éthyle, propyle, butyle, pentyle, hexyle, heptyle, octyle et hydroxyméthyle.

7. Procédé de préparation d'une composition (C) telle que définie à l'une des revendications 1 à 6, comprenant les étapes successives de réactions chimiques suivantes :

une étape a) de réaction d'un alcool de formule (IV) :

R1-OH (IV)

dans laquelle R1 représente un radical aliphatique saturé ou insaturé, linéaire ou ramifié, comportant de 8 à 36 atomes de carbone, éventuellement substitué avec un ou plusieurs groupes hydroxyle ou d'un mélange d'alcools de formule (IV), avec un oxétane- 3, 3 - disubstitué de formule (V) :

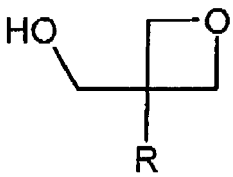

(V)

dans laquelle R représente un radical alkyle linéaire ou ramifié comportant de un à huit atomes de carbone éventuellement substitué par un ou plusieurs radicaux hydroxy, conduisant à un mélange (M) comprenant l'alcool de formule (IV) n'ayant pas réagit et un ou plusieurs composés choisis parmi les composés de formules (IIa), (IIb), ($IIc_1$) ou ($IIc_2$) :

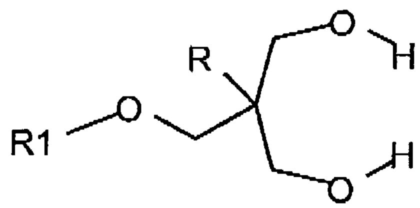

(IIa)

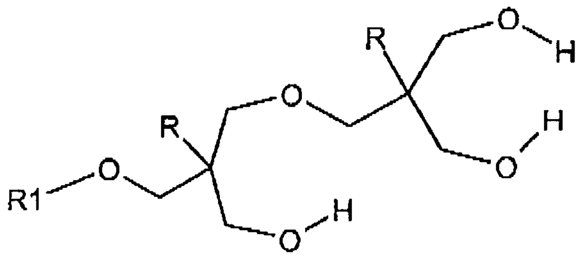

(IIb)

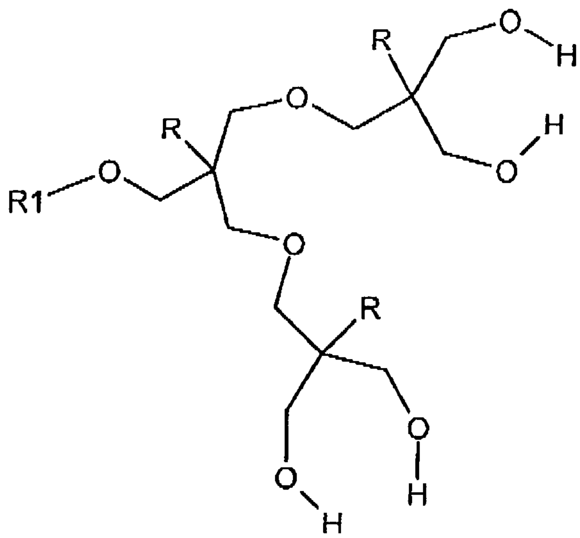

(IIc1)

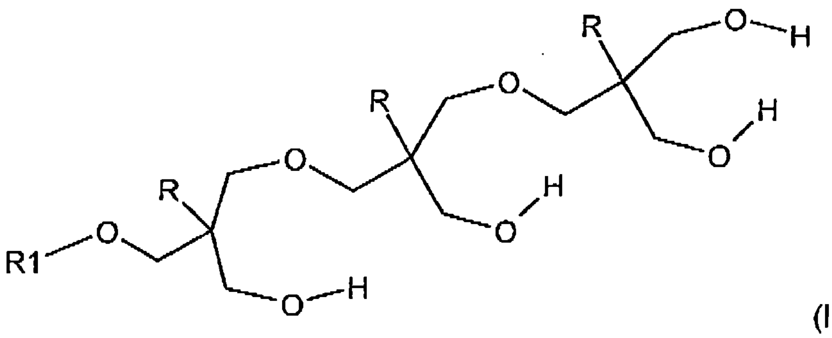

(IIc2)

dans lesquelles R et R1 sont tels que définis précédemment;
une étape b) de réaction dudit mélange (M) avec un sucre réducteur de formule (III) :

S-H (III)

pour obtenir ladite composition (C).

8. Procédé tel que défini à la revendication 7, comprenant en outre une étape c) de déshydratation d'un composé de formule (VII) :

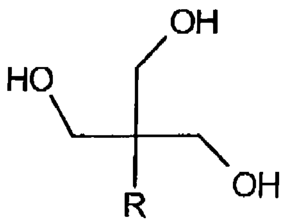

(VII)

dans laquelle R est tel que défini précédemment, pour obtenir le composé de formule (V).

9. Utilisation d'une composition (C) telle que définie à l'une des revendications 1 à 6, comme agent tensioactif et plus particulièrement, comme agent moussant, agent émulsionnant, agent mouillant, agent dispersant ou agent détergent.

10. Composition cosmétique ou pharmaceutique **caractérisée en ce qu'**elle comprend comme agent tensioactif, une composition (C) telle que définie à l'une des revendications 1 à 6.

## Claims

1. Composition (C) comprising per 100% of its mass:

- from 0.5% to 30% of one or more alcohols of formula (IV):

R1-OH (IV)

in which R1 represents a saturated or unsaturated, linear or branched aliphatic radical having from 8 to 36 carbon atoms, optionally substituted with one or more hydroxyl groups,
and
- from 70% to 99.5% of one or more compounds of formula (I) chosen from:

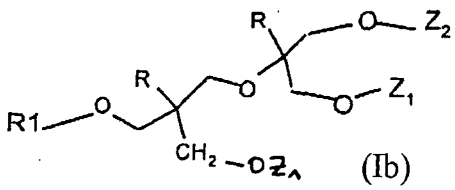

(Ib)

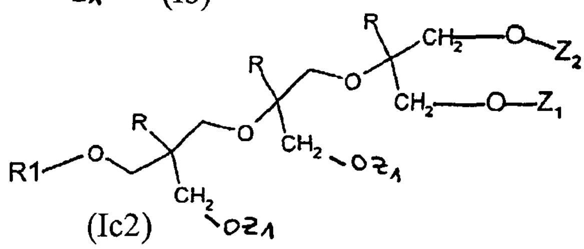

(Ic2)

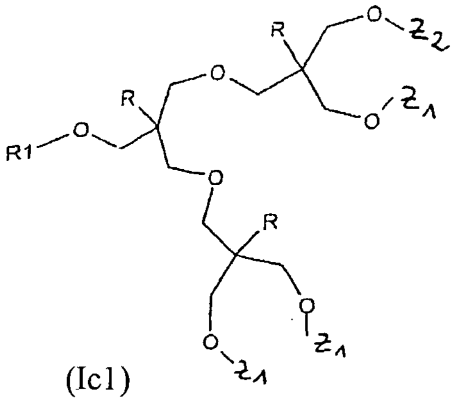

(Ic1)

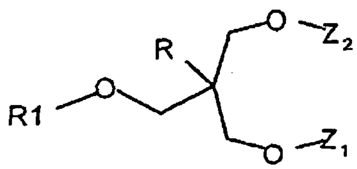

(Ia)

where R represents a linear or branched alkyl radical having from one to eight carbon atoms optionally substituted with one or more hydroxyl radicals, R1 is as defined previously for formula (IV), at least one of the radicals $Z_1$ or $Z_2$ represents a radical:

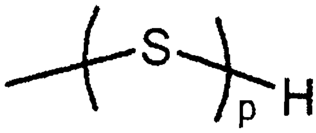

in which -S- represents the divalent residue of a reducing sugar and p represents a decimal number greater than or equal to 1 and less than or equal to 10 and the other one of the radicals $Z_1$ or $Z_2$ represents a hydrogen atom or said radical:

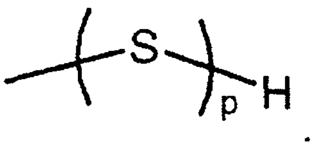

2. Composition (C) as defined in Claim 1, for which R1 represents a radical selected from the radicals octyl, decyl, dodecyl, tetradecyl, hexadecyl, octadecyl, eicosyl, 2-ethylhexyl, 2-butyloctyl, 2-butyldecyl, 2-hexyloctyl, 2-hexyldecyl, 2-hexyldodecyl, 2-octyldecyl, 2-octyldodecyl, 2-decyltetradecyl, isododecyl, isotetradecyl, isohexadecyl, isooctadecyl, 10-undecenyl, oleyl, isooleyl, linoleyl, linolenyl or 10,13-eicosadienyl, 12-hydroxystearyl and 2-propylheptyl.

3. Composition (C) as defined in either of Claims 1 and 2, where -S- represents a divalent glucose residue.

4. Composition (C) as defined in either of claims 1 and 2, for which in formula (I), S represents a divalent xylose residue.

5. Composition (C) as defined in one of Claims 1 to 4, for which p is between 1.005 and 5 and more particularly between 1.05 and 2.

**6.** Composition (C) as defined in one of Claims 1 to 4, for which R is selected from the methyl, ethyl, propyl, butyl, pentyl, hexyl, heptyl, octyl and hydroxymethyl radicals.

**7.** Method of preparation of a composition (C) as defined in one of Claims 1 to 6, comprising the following successive stages of chemical reactions:

a stage a) of reaction of an alcohol of formula (IV):

R1-OH (IV)

in which R1 represents a saturated or unsaturated, linear or branched aliphatic radical having from 8 to 36 carbon atoms, optionally substituted with one or more hydroxyl groups or of a mixture of alcohols of formula (IV), with a 3,3-disubstituted oxetane of formula (V):

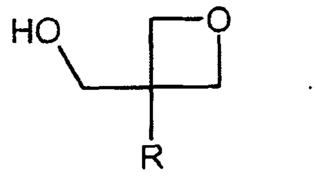

(V)

in which R represents a linear or branched alkyl radical having from one to eight carbon atoms optionally substituted with one or more hydroxyl radicals, leading to a mixture (M) comprising the alcohol of formula (IV) that had not reacted and one or more compounds chosen from the compounds of formula (IIa) , (IIb) , ($IIc_1$) or ($IIc_2$) :

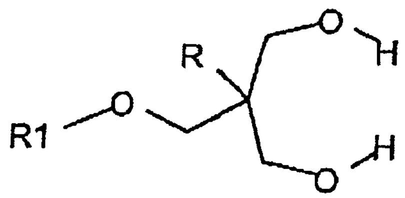

(IIa)

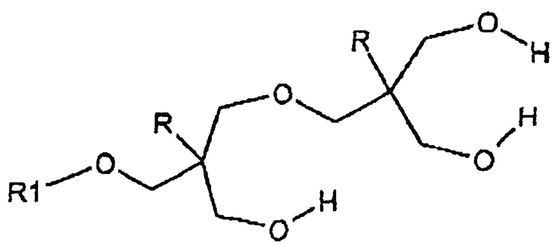

(IIb)

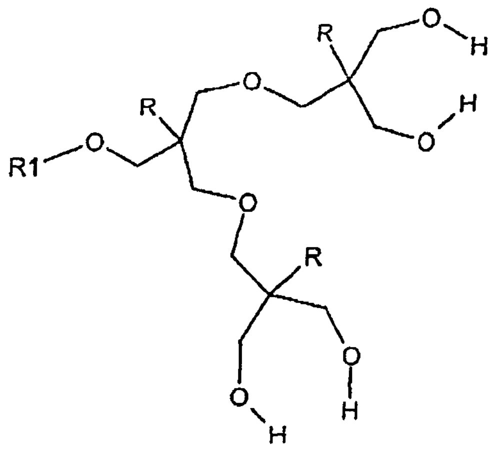

(IIc1)

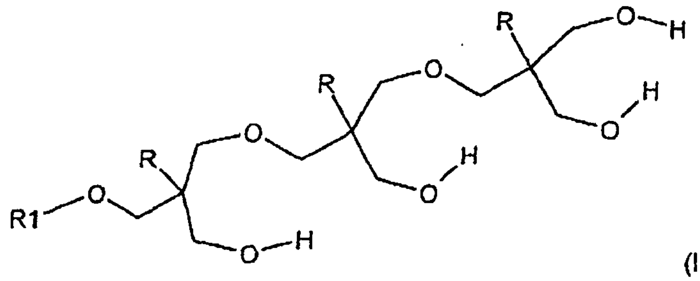

(IIc2)

in which R and R1 are as defined previously;
a stage b) of reaction of said mixture (M) with a reducing sugar of formula (III):

S-H (III)

to obtain said composition (C).

**8.** Method as defined in Claim 7, additionally comprising a stage c) of dehydration of a compound of formula (VII):

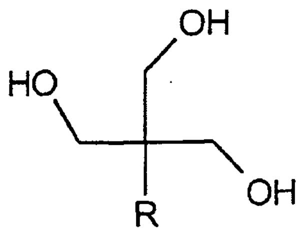

(VII)

in which R is as defined previously, to obtain the compound of formula (V).

**9.** Use of a composition (C) as defined in one of Claims 1 to 6, as a surfactant and more particularly as a foaming agent, emulsifier, wetting agent, dispersant or detergent.

**10.** Cosmetic or pharmaceutical composition, **characterized in that** it contains, as surfactant, a composition (C) as defined in one of Claims 1 to 6.

**Patentansprüche**

1. Zusammensetzung (Z), enthaltend pro 100% seiner Masse:

   - 0,5% bis 30% eines oder mehrerer. Alkohole der Formel (IV):

   R1-OH (IV),

   worin R1 für einen gesättigten oder ungesättigten, linearen oder verzweigten Rest, der 8 bis 36 Kohlenstoffatome enthält und gegebenenfalls durch eine oder mehrere Hydroxylgruppen substituiert ist, steht,
   und
   - 70% bis 99,5% einer oder mehrerer Verbindungen der Formel (I), ausgewählt unter:

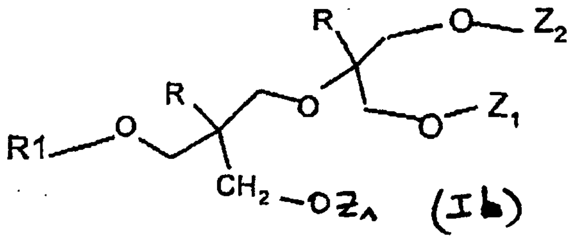

(Ib)

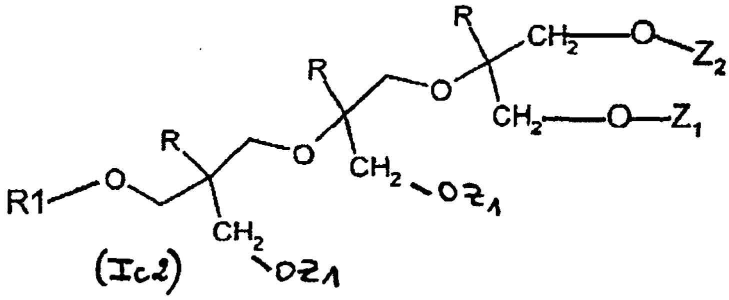

(Ic2)

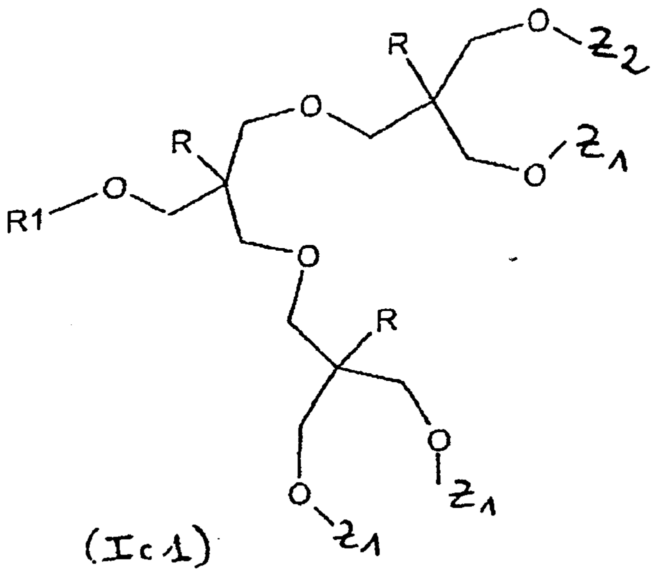

(Ic1)

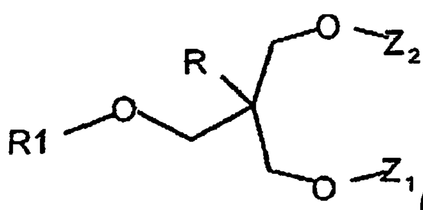

(I a)

worin R für einen linearen oder verzweigten Alkylrest, der eins bis acht Kohlenstoffatome enthält und gegebenenfalls durch einen oder mehrere Hydroxyreste substituiert ist, steht, R1 die oben für die Formel (IV) angegebene Bedeutung besitzt, mindestens einer der Reste $Z_1$ und $Z_2$ für einen Rest:

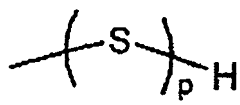

worin -S- für einen zweiwertigen Rest eines reduzierenden Zuckers steht und p für eine Dezimalzahl größer gleich 1 und kleiner gleich 10 steht, und der andere der Reste $Z_1$ und $Z_2$ für ein Wasserstoffatom oder den Rest

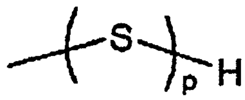

steht.

**2.** Zusammensetzung (Z) nach Anspruch 1, für die R1 für einen unter den Resten Octyl, Decyl, Dodecyl, Tetradecyl, Hexadecyl, Octadecyl, Eicosyl, 2-Ethylhexyl, 2-Butyloctyl, 2-Butyldecyl, 2-Hexyloctyl, 2-Hexyldecyl, 2-Hexyldodecyl, 2-Octyldecyl, 2-Octyldodecyl, 2-Decyltetradecyl, Isododecyl, Isotetradecyl, Isohexadecyl, Isooctadecyl, 10-Undecenyl, Oleyl, Isooleyl, Linoleyl, Linolenyl oder 10,13-Eicosadienyl, 12-Hydroxystearyl und 2-Propylheptyl ausgewählten Rest steht.

**3.** Zusammensetzung (Z) nach einem der Ansprüche 1 oder 2, wobei -S- für einen zweiwertigen Glucoserest steht.

**4.** Zusammensetzung (Z) nach einem der Ansprüche 1 oder 2, für die in Formel (I) S für einen zweiwertigen Xyloserest steht.

**5.** Zusammensetzung (Z) nach einem der Ansprüche 1 bis 4, für die p zwischen 1,005 und 5 und insbesondere zwischen 1,05 und 2 liegt.

**6.** Zusammensetzung (Z) nach einem der Ansprüche 1 bis 4, für die R unter den Resten Methyl, Ethyl, Propyl, Butyl, Pentyl, Hexyl, Heptyl, Octyl und Hydroxymethyl ausgewählt ist.

**7.** Verfahren zur Herstellung einer Zusammensetzung (Z) nach einem der Ansprüche 1 bis 6 mit den folgenden aufeinanderfolgenden Stufen chemischer Reaktionen:

einer Stufe a) der Umsetzung eines Alkohols der Formel (IV):

R1-OH (IV)

worin R1 für einen gesättigten oder ungesättigten, linearen oder verzweigten Rest, der 8 bis 36 Kohlenstoffatome enthält und gegebenenfalls durch eine oder mehrere Hydroxylgruppen substituiert ist, steht, oder ein Gemisch von Alkoholen der Formel (IV) mit einem 3,3-disubstituierten Oxetan der Formel (IV):

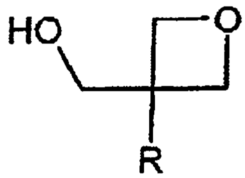

(V)

worin R für einen linearen oder verzweigten Alkylrest, der eins bis acht Kohlenstoffatome enthält und gegebenenfalls durch einen oder mehrere Hydroxyreste substituiert ist, steht, was ein Gemisch (G) ergibt, das nicht umgesetzten Alkohol der Formel (IV) und eine oder mehrere Verbindungen, die unter den Verbindungen der Formeln (IIa), (IIb), ($IIc_1$) oder ($IIc_2$) ausgewählt sind, enthält:

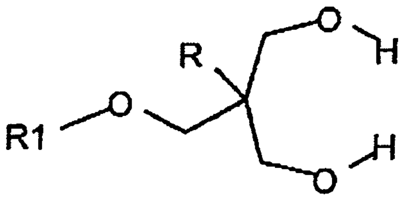

(IIa)

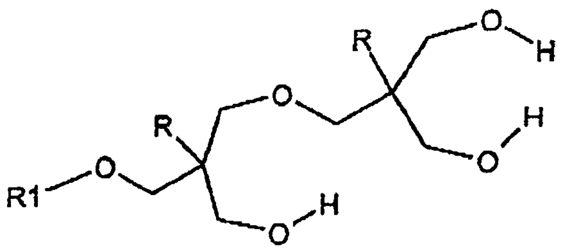

(IIb)

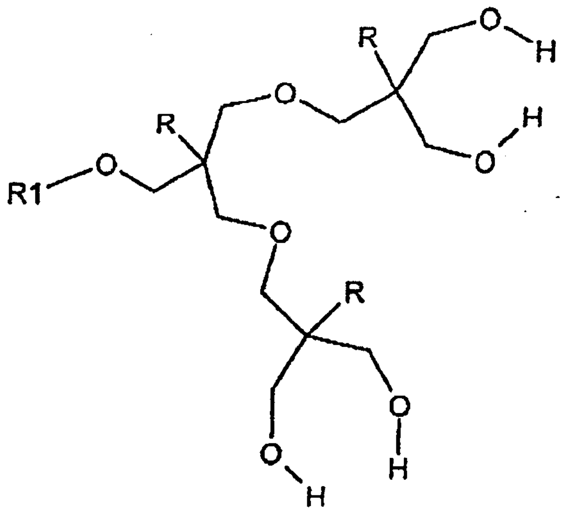

(IIc1)

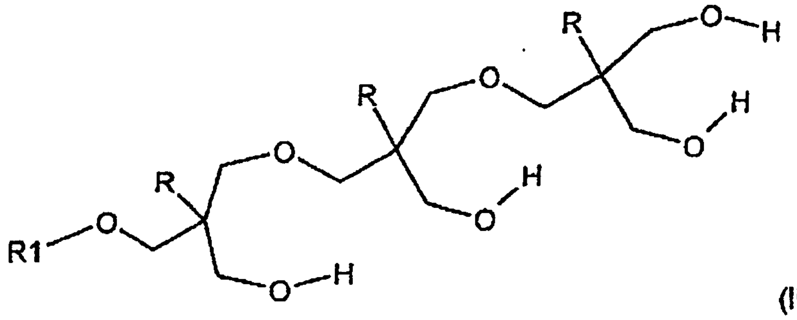

(IIc2)

worin R und R1 die oben angegebene Bedeutung besitzen;
einer Stufe b) der Umsetzung des Gemischs (G) mit einem reduzierenden Zucker der Formel (III):

S-H (III)

wobei man die Zusammensetzung (Z) erhält.

**8.** Verfahren nach Anspruch 7 mit einer weiteren Stufe c) der Dehydratisierung einer Verbindung der Formel (VII):

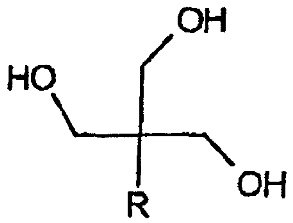

(VII)

worin R die oben angegebene Bedeutung besitzt, wobei man die Verbindung der Formel (V) erhält.

**9.** Verwendung einer Zusammensetzung (Z) nach einem der Ansprüche 1 bis 6 als Tensid und insbesondere als Schäumungsmittel, Emulgator, Netzmittel, Dispergiermittel oder Detergens.

**10.** Kosmetische oder pharmazeutische Zusammensetzung, **dadurch gekennzeichnet, daß** sie als Tensid eine Zusammensetzung (Z) nach einem der Ansprüche 1 bis 6 enthält.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- DE 19728900
- FR 2804432